(19) Europäisches Patentamt
European Patent Office
Office européen des brevets

(11) Veröffentlichungsnummer: **0 274 700 B1**

(12) **EUROPÄISCHE PATENTSCHRIFT**

(45) Veröffentlichungstag der Patentschrift: **31.05.95**

(51) Int. Cl.[6]: **C07D 265/38**, C07H 17/00, C12Q 1/34, C12Q 1/42

(21) Anmeldenummer: **87118742.3**

(22) Anmeldetag: **17.12.87**

# (54) Neue Hydrolase-Substrate.

(30) Priorität: **24.12.86 DE 3644401**

(43) Veröffentlichungstag der Anmeldung:
**20.07.88 Patentblatt 88/29**

(45) Bekanntmachung des Hinweises auf die Patenterteilung:
**31.05.95 Patentblatt 95/22**

(84) Benannte Vertragsstaaten:
**AT BE CH DE ES FR GB GR IT LI LU NL SE**

(56) Entgegenhaltungen:
**EP-A- 0 156 347**
**EP-A- 0 157 384**
**EP-A- 0 209 875**
**EP-A- 0 210 559**
**EP-A- 0 217 371**

(73) Patentinhaber: **BOEHRINGER MANNHEIM GMBH**

**D-68298 Mannheim (DE)**

(72) Erfinder: **Von Der Eltz, Herbert, Dr. rer. nat.**
**In der Au 21**
**D-8120 Weilheim (DE)**
Erfinder: **Guder, Hans-Joachim, Dr. rer. nat.**
**Schwattachweg 1**
**D-8120 Weilheim (DE)**
Erfinder: **Mühlegger, Klaus**
**Römerstrasse 7**
**D-8121 Polling (DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

Die vorliegende Erfindung betrifft Dihydroresorufin-Verbindungen, welche als chromogene und/oder fluorogene Substrate für Hydrolasen, geeignet sind, Verfahren zu ihrer Herstellung sowie ein entsprechendes Reagenz zur Bestimmung von Hydrolasen.

Unter Hydrolasen werden allgemein Enzyme verstanden, welche Bindungen unter Wasserverbrauch, hydrolytisch, spalten. In der klinischen Chemie und in der Diagnostik hat in den letzten Jahren vor allem die Bestimmung der Aktivität solcher Hydrolasen an Bedeutung gewonnen, welche Ester- und Etherbindungen spalten. Zu nennen sind hier beispielhaft: Esterasen, wie z. B. die in Leucozyten vorkommenden Carboxylester spaltenden Enzyme oder Phosphatasen, wie z. B. alkalische oder saure Phosphatasen, die Phosphorsäureester hydrolisieren. Glykosidasen, wie z. B. Galactosidasen, Glucosidasen, Mannosidasen oder Amylasen spalten glykosidische Bindungen.

Zur Bestimmung der Aktivität von Hydrolasen wird die enzymhaltige Probe mit einem geeigneten Substrat versetzt. Das Substrat wird von dem Enzym gespalten und eines der Spaltprodukte wird in geeigneter Weise nachgewiesen. Als Substrat eignen sich oft die natürlichen Substrate der nachzuweisenden Enzyme. Besonders bevorzugt werden jedoch chromogene oder fluorogene Verbindungen verwendet, bei denen eines der Spaltprodukte einen Rest darstellt, der spektroskopisch im sichtbaren oder im UV-Bereich oder fluorimetrisch nachgewiesen werden kann.

Bisher bekannte Hydrolase-Substrate besitzen als Chromophor z. B. Phenol, Naphthol oder Derivate hiervon. Solche Chromophore absorbieren im sichtbaren Wellenlängenbereich überhaupt nicht oder nur schwach. UV-spektrometrisch sind sie im kurzwelligen Bereich meßbar. Da die die zu messenden Enzyme enthaltenden Proben meist, bedingt durch weitere Inhaltsstoffe, erhebliche Eigenabsorption in diesen Wellenlängenbereichen aufweisen, können die Messungen stark gestört werden. Um solche Störungen zu vermeiden, können die durch Einwirkung einer Hydrolase zunächst entstehenden Spaltprodukte, wie Phenole oder Naphthole z. B. mit Aminoantipyrrin oxidative gekuppelt oder mit verschiedenen Diazoniumsalzen zu Azofarbstoffen umgesetzt und die so entstehenden Farbstoffe dann gemessen werden.

Die Bestimmung von Enzymaktivitäten mit fluorogenen Substraten ist weit verbreitet, da gegenüber den photometrischen Methoden die Empfindlichkeit fluorometrischer Bestimmungen oft um einige Zehnerpotenzen erhöht ist. In manchen Fällen muß mit fluorogenen Substraten gearbeitet werden, beispielsweise bei der Untersuchung enzymatischer Aktivität in Zellen mit automatischen Geräten zur Zelldifferenzierung (Zytofluorometrie) sowie bei der Analyse immobilisierter Enzyme mit Durchflußmikrofluorometrie. In anderen Fällen, beispielsweise bei der Bestimmung enzymatischer Markierung von Testsystemen (Enzymimmunoassays) wird der Multiplikationseffekt der enzymatischen Katalyse durch Verwendung fluorogener Substrate beträchtlich verstärkt.

Bisher bekannte fluorogene Substrate für Hydrolasen besitzen als Fluorophore z. B. Fluorescein, Indoxyl, Methylumbelliferron oder Derivate dieser Verbindungen. Für die kinetische Analyse komplexer Systeme besitzen diese Verbindungen jedoch schwerwiegbende Nachteile. So fluoreszieren die als Hydrolase-Substrate eingesetzten Fluorescein-Derivate bereits oft schon selbst. Indoxyl-Derivate unterliegen nach ihrer enzymatischen Spaltung einer Reihe von chemischen Umwandlungen, die ebenfalls die kinetische Analyse komplizieren. Derivate von Methylumbelliferron müssen im UV angeregt werden. Hierbei kann die Eigenfluoreszenz von biologischen oder synthetischen Materialien stören. Darüber hinaus ist die UV-Anregung insbesondere bei Laseroptik, kostenaufwendig. Die meisten fluorogenen Substrate führen zu Reaktionsprodukten, die nur eine geringe Löslichkeit aufweisen, so daß sie für die kinetische Analyse von Enzymaktivitäten, wofür gerade gute Löslichkeit von Substrat und Produkt erforderlich ist, nicht geeignet sind.

Als spezielle Glykosidase-Substrate sind aus DE-OS 34 11 574 Resorufin-Glykoside bekannt. Diese sind in wässriger Lösung meist gelb gefärbt. Nach der enzymatischen Reaktion weisen die Produkte eine rote Farbe auf.

In der DE-OS-34 115 74, der EP-A-0 210 559 und der EP-A-0 217 371 sind Triacylresorufine als Ausgangsstoffe zur chemischen Synthese komplexer Resorufine beschrieben. Die Eignung dieser Verbindungen als Substrate für Hydrolasen ist nicht beschrieben.

Trotz der Vielfalt bekannter chromogener oder fluorogener Substrate zum Nachweis von Hydrolasen wird immer noch nach Verbindungen gesucht, die breit, d. h. für den Nachweis unterschiedlichster Hydrolasen einsetzbar sind, eine hohe Empfindlichkeit besitzen und für die verschiedensten Verfahren, wie z. B. UV-photometrische, visuelle und fluorimetrische Messungen gut geeignet sind.

Aufgabe der Erfindung war es, farblose und wasserlösliche Hydrolase-Substrate bereitzustellen, welche in einer zweistufigen Reaktion in farbige und fluoreszierende Verbindungen überführt werden können.

Gelöst wird diese Aufgabe durch die neuen erfindungsgemäßen Verbindungen, die durch Hydrolasen in einen Säure- oder Hydroxyanteil und in ein Dihydroresorufin-Derivat mit mindestens einer freien OH-Gruppe gespalten werden. Letztere sind gut wasserlösliche Verbindungen, die leicht zu Resorufin-Verbindungen oxidiert werden können, welche eine gut meßbare Absorption im sichtbaren Bereich aufweisen und sich ferner leicht zur Fluoreszenz anregen lassen.

Gegenstand der vorliegenden Erfindung sind demnach farblose und wasserlösliche Verbindungen der allgemein Formel I

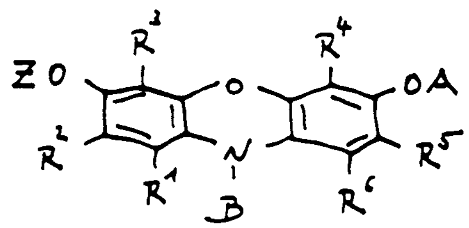

(I)

in der

| | |
|---|---|
| Z | einen organischen oder anorganischen Säurerest oder einen Zuckerrest, |
| A | einen organischen oder anorganischen Säurerest, |
| B | einen organischen Säurerest und |
| $R^2$ und $R^5$, | die gleich oder verschieden sein können, Wasserstoff Halogen oder eine $C_1$-$C_5$-Alkylgruppe, |
| $R^1$, $R^3$, $R^4$ und $R^6$, | die gleich oder verschieden sein können, Wasserstoff, Halogen, eine Cyano-, $C_1$-$C_5$-Alkyl-, $C_1$-$C_5$Alkoxy-, Carboxy-, $C_1$-$C_5$-Alkoxycarbonyl-, Carboxy-$C_1$-$C_5$-alkyl-, $C_1$-$C_5$Alkoxycarbonyl-$C_1$-$C_5$-Alkyl- oder eine gegebenenfalls ein- oder zweifach substituierte Carboxamidogruppe oder die Gruppe<br><br>$-COO-(CH_2CH_2O)_n-R^7$<br><br>in der |
| $R^7$ | Wasserstoff oder eine $C_1$-$C_5$-Alkyl-Gruppe und n eine ganze Zahl von 1 - 4 bedeuten,<br>wobei |
| $R^6$ | zusätzlich eine Sulfo- oder Nitrogruppe vorstellen kann, |

und wobei

a) A, B und Z nicht gleichzeitig eine identische Acylgruppe sind

oder

b) A ungleich Z und A gleich B, oder
A gleich Z und A ungleich B, oder
A, B und Z alle ungleich sind,

bedeuten.

Unter einem anorganischen Säurerest in der Definition von A und Z sind vor allem der Ortho- oder Pyrophosphorsäure- oder Schwefelsäurerest zu verstehen, die über eine Esterbindung an das Dihydroresorufingrundgerüst gebunden sind. Bevorzugt sind die Reste $PO_3MM'$ und $SO_3M$, insbesondere $PO_3MM'$, wobei im Falle der freien Säuren M und M' Wasserstoff bedeuten, während wenn die Säuren als Salz vorliegen, M und M' für Alkali-, Erdalkali- oder Ammoniumionen stehen.

Unter Alkaliionen in der Definition von M und M' sind vor allem Lithium-, Natrium- und Kaliumionen zu verstehen. Erdalkaliionen sind vor allem Magnesium-, Calcium- oder Bariumionen.

Ammoniumionen in der Definition von M und M' können das unsubstituierte Ammoniumion oder durch $C_1$-$C_5$-Alkyl- oder Aryl-$C_1$-$C_5$-alkylreste ein- oder mehrfach substituierte Ammoniumionen sein.

Bevorzugt als $C_1$-$C_5$-Alkylrest sind der Methyl- oder Ethylrest. Unter einem Aryl-$C_1$-$C_5$-alkylrest ist vor allem der Benzylrest zu verstehen. Die Substituenten substituierter Ammoniumionen können sowohl gleich als auch verschieden sein.

Unter einem organischen Säurerest in der Definition von Z werden vor allem Alkancarbonsäure-, Aminosäure- oder Oligopeptidreste verstanden, welche mit ihrem Carboxylende als Ester an den Dihydroresorufin-Kern gebunden vorliegen.

Alkancarbonsäuren in der Definition von Z sind Verbindungen mit 1 - 20 Kohlenstoffatomen. Besonders bevorzugt sind Essigsäure, Propionsäure, Buttersäure, Palmitinsäure und Stearinsäure. Neben gesättigten Säureresten kann Z auch ein ungesättigter Säurerest, wie z. B. der Ölsäure-, Linolsäure- oder Linolensäurerest sein.

Als Aminosäurerest kommen vorzugsweise die Reste der natürlichen $\alpha$-Aminosäuren in ihrer L- oder D-Form oder auch in ihrer racemischen Form in Frage. Besonders bevorzugt sind die Reste von Glycin, Alanin, Valin, Leucin, Isoleucin, Phenylalanin und Thyrosin, wobei jeweils die L-Form ganz besonders bevorzugt ist. Gegebenenfalls vorhandene freie Hydroxygruppen können acyliert, vorzugsweise acetyliert, sein.

Unter einem Oligopeptidrest sind z. B. Di-, Tri-, Tetra- und Pentapeptide, vorzugsweise Di- und Tripeptide zu verstehen, wobei als Aminosäurekomponenten vorzugsweise die oben erwähnten Aminosäuren Verwendung finden.

Die Aminogruppen der esterartig an den Dihydroresorufin-Kern gebundenen Aminosäure- oder Oligopeptidreste können frei oder geschützt vorliegen. Als Schutzgruppe sind hier alle üblichen Aminoschutzgruppen zu verstehen, insbesondere Acyl-, Oxycarbonyl-, Thiocarbonyl-, Sulfo-, Sulfino-, Vinyl-, Cyclohexenyl-, Phosphoryl- oder Carbamoyl-Gruppen. Besonders bevorzugte Aminoschutzgruppen sind der Tosyl-, Benzyloxycarbonyl- und tert.-Butoxycarbonyl-Rest.

In der Definition von B bedeutet ein organischer Säurerest vor allem eine gegebenenfalls durch Hydroxyl-, Carboxyl- und/oder einen Ammoniumrest ein- oder mehrfach substituierte $C_1$-$C_7$-Alkylcarbonylgruppe. Der $C_1$-$C_7$-Alkylrest der $C_1$-$C_7$-Alkylcarbonylgruppe ist ein Kohlenwasserstoffrest aus vorzugsweise 1 - 5 Kohlenstoffatomen. Er kann gesättigt oder ungesättigt, geradkettig oder verzweigt sein.

Ammoniumreste sind Reste $-NR_3^+$ , wobei R Wasserstoff, ein $C_1$-$C_5$-Alkyl- oder Aryl-$C_1$-$C_5$-alkylrest sein kann. Grundsätzlich gilt für die möglichen Ammoniumreste das gleiche wie für die Ammoniumionen in der Definition von M und M'. Ein $C_1$-$C_5$-Alkylrest ist bevorzugt Methyl oder Ethyl. Ein bevorzugter Aryl -$C_1$-$C_5$-alkylrest ist der Benzylrest. Die Substituenten R möglicher Ammoniumreste können sowohl gleich als auch verschieden sein. Ein besonders bevorzugter Ammoniumrest ist $-N(CH_3)_3^+$ .

Besonders bevorzugte Säurereste in der Definition von B sind der Acetyl-, Oxalyl-, Malonyl-, Succinyl-, Glutaryl- oder Carnityl-Rest.

Ein organischer Säurerest in der Definition von A umfaßt die Bedeutungen von B und Z. A kann ein Alkancarbonsäurerest oder ein Aminosäure- oder Oligopeptid-Rest sein oder eine gegebenenfalls durch Hydroxyl-, Carboxyl- und/oder Ammoniumreste ein- oder mehrfach substituierte $C_1$-$C_7$-Alkylcarbonyl-Gruppe. Die einzelnen Definitionen für die organischen Säurereste B oder z gelten auch für den organischen Säurerest in der Definition von A. Verbindungen der allgemeinen Formel I, in denen A einen organischen Säurerest darstellt, sind besonders bevorzugt, wenn A die gleiche Bedeutung wie B hat.

Ein Zuckerrest in der Definition von Z kann ein Mono- oder Oligosaccharid sein. Der Zuckerrest kann $\alpha$- oder $\beta$-glycosidisch an das Dihydroresorufin-Grundgerüst gebunden sein. Beispiele für bevorzugte Monosaccharide sind Galactose, Glucose oder Mannose.

Als Zuckerreste sind aber auch Oligosaccharide geeignet. Als Oligosaccharide werden insbesondere solche bezeichnet, die aus 2 bis 10, vorzugsweise aus 2 - 7 Monosaccharideinheiten aufgebaut sind. Besonders bevorzugt sind Heptaosen.

In der Definition der Reste $R^1$ - $R^7$ sind unter Halogen Fluor, Chlor, Brom und Jod, vorzugsweise Chlor und Brom, zu verstehen.

Die $C_1$-$C_5$-Alkylgruppe in der Definition von $R^1$ - $R^7$ enthält vorzugsweise 1 - 3 Kohlenstoffatome, wobei die Methylgruppe besonders bevorzugt ist.

Die $C_1$-$C_5$-Alkoxygruppe in der Definition von $R^1$, $R^3$, $R^4$ und $R^6$ enthält vorzugsweise 1 - 3 Kohlenstoffatome, wobei die Methoxygruppe besonders bevorzugt ist.

Die $C_1$-$C_5$-Alkoxy- bzw. die $C_1$-$C_5$-Alkylreste der $C_1$-$C_5$-Alkoxycarbonyl-, Carboxy -$C_1$-$C_5$-alkyl- sowie der $C_1$-$C_5$-Alkoxycarbonyl-$C_1$-$C_5$-alkyl- Reste in der Definition der Substituenten $R^1$, $R^3$, $R^4$ und $R^6$ weisen ebenfalls 1 - 5, vorzugsweise 1 - 3 Kohlenstoffatome auf, wobei die Methoxy-Gruppe bzw. der Methyl-Rest besonders bevorzugt sind.

Als Substituenten der Carboxamido-Gruppe kommen Alkyl-, Alkoxyalkyl-, Carboxyalkyl- und Alkoxycarbonylalkyl-Reste in Frage, wobei die Alkyl- oder Alkoxy-Reste jeweils 1 - 5, vorzugsweise 1 - 3 Kohlenstoffatome aufweisen. Bei einer zweifach substituierten Carboxamidfunktion können die beiden Substituenten zu einem Ring geschlossen sein, der durch Heteroatome, z. B. Sauerstoff, Stickstoff und Schwefel, unterbrochen sein kann. Besonders bevorzugt in diesem Sinne ist der Morpholin-Rest als Aminteil der Carboxamidogruppe.

Bevorzugte Verbindungen der allgemeinen Formel I sind solche, wobei

Z $PO_3MM'$, $SO_3M$, einen carboxylgebundenen Alkancarbonsäure-, Aminosäure- oder Oligo-

| | |
|---|---|
| | peptidrest, einen Zuckerrest, |
| A | $PO_3MM'$, $SO_3M$, einen $C_1$-$C_5$-alkylcarbonyl-Rest, der gegebenenfalls durch Carboxyl-, Hydroxyl- und/oder Ammoniumreste ein- oder mehrfach substituiert ist oder einen carboxylgebundenen Aminosäure- oder Oligopeptidrest, |
| M und M' | Wasserstoff, ein Alkali-, Erdalkali- oder Ammoniumion und |
| B | einen gegebenenfalls durch Carboxyl-, Hydroxyl- und/oder Ammoniumreste ein- oder mehrfach substituierten $C_1$-$C_7$-Alkylcarbonyl-Rest |

bedeuten sowie

$R^1$ - $R^7$ die oben angegebene Bedeutung haben.

Verbindungen der allgemeinen Formel I sind neu. Sie können nach an sich bekannten Methoden aus Resorufinen der allgemeinen tautomeren Formeln II a und II b

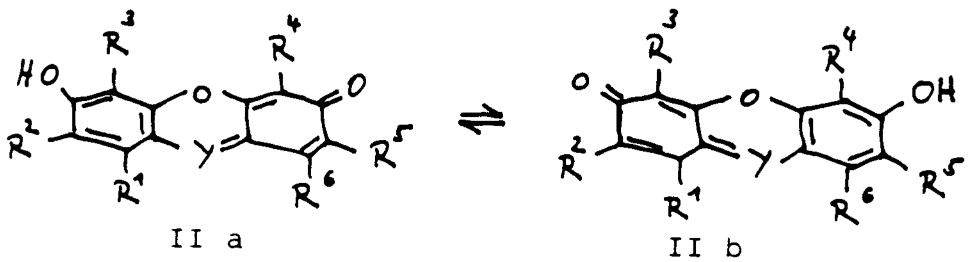

II a　　　II b

in denen

| | |
|---|---|
| $R^1$ - $R^6$ | die für Verbindungen der allgemeinen Formel I angegebene Bedeutung haben und |
| Y | Stickstoff oder die Gruppe N -> O |

bedeuten,

hergestellt werden. Je nachdem, ob

a) A ungleich Z und A gleich B,

b) A gleich Z und A ungleich B ist oder

c) A, B und Z alle ungleich sind,

können drei Varianten unterschieden werden.

Zur Synthese von Verbindungen der allgemeinen Formel I, in der A ungleich Z und A gleich B ist, werden Verbindungen der allgemeinen tautomeren Formeln II a und II b mit einer Verbindung der allgemeinen Formel III

X - Z　　(III)

wobei

| | |
|---|---|
| Z | die in der allgemeinen Formel I angegebene Bedeutung hat und |
| X | eine reaktive Gruppe |

bedeutet,

zu einem Resorufin-Derivat der tautomeren Formeln IV a und IV b

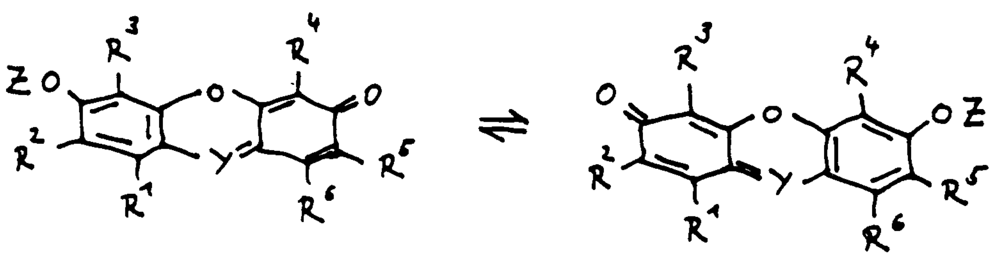

IV a　　　IV b

in denen

$R^1$ - $R^6$, Y und Z die oben angegebene Bedeutung haben,

umgesetzt, reduziert und mittels einer Berbindung der allgemeinen Formel V

X-B (V)

wobei

B die für Verbindungen der allgemeinen Formel I angegebene Bedeutung hat und

X eine reaktive Gruppe

bedeutet, acyliert.

Resorufine der allgemeinen Formeln II a bzw. II b sind Gegenstand der europäischen Patentanmeldung EP-A-0 156 347.

Im Acylierungsreagenz der allgemeinen Formel III hat Z die für Verbindungen der allgemeinen Formel I angegebene Bedeutung. Vorzugsweise handelt es sich um einen anorganischen Säurerest aus der Gruppe der Orthophosphorsäure oder Schwefelsäure, einen Alkancarbonsäure-, Aminosäure- oder Oligopeptidrest oder um einen Zuckerrest. Freie Amino- oder Hydroxygruppen von Aminosäuren-, Oligopeptid- oder Zuckerresten können hierbei gegebenenfalls durch entsprechende übliche Schutzgruppen geschützt sein, die später nach Durchführung des letzten Acylierungsschrittes mit X-A unter geeigneten Bedingungen wieder abgespalten werden können.

X bedeutet eine reaktive Gruppe, die in der Lage ist, mit der phenolischen OH-Gruppe des Resorufins der allgemeinen Formel II a und II b eine Reaktion einzugehen. Die Wahl der reaktiven Gruppe X ist abhängig von der Natur des Restes Z. Handelt es sich bei Z um einen Zuckerrest, so ist X vorzugsweise ein Acetylrest oder ein Halogenatom, welches aus der Gruppe Fluor, Chlor, Brom und Jod ausgewählt werden kann. Bevorzugt werden Chlor, Brom und Jod.

Die Synthese von Resorufinglycosiden der allgemeinen Formeln IV a und IV b, in denen Z einen Zuckerrest bedeutet, kann gemäß der EP-A-0 156 347 erfolgen.

Wenn Z ein Aminosäure- oder Peptidrest bedeutet, der mit seinem Carboxylende mit einem Resorufin der allgemeinen Formeln II a und II b verestert werden soll, dann kommen als reaktive Gruppe X die in der Peptidchemie üblichen Gruppen in Frage. Als reaktive Derivate werden z. B. die Säurehalogenide, bevorzugt Säurechloride bzw. die bei der Peptidsynthese üblicherweise verwendeten Mischanhydride oder Aktivester eingesetzt. Die gleichen reaktiven Gruppen können auch für die Bindung von Alkancarbonsäuren an das Resorufin-Gerüst eingesetzt werden.

Für den Fall, daß Z einen anorganischen Säurerest bedeutet, werden Resorufine der allgemeinen Formeln II a und II b vorzugsweise mit den entsprechenden Säurehalogeniden, insbesondere Säurechloriden umgesetzt.

Für die Synthese von Verbindungen der allgemeinen Formel IV a und IV b, in denen Z den Rest $PO_3MM'$ bedeutet, kann nach dem in der deutschen Offenlegungsschrift 35 34 927 beschriebenen Verfahren vorgegangen werden.

Die Reduktion der Resorufin-Derivate der allgemeinen Formeln IV a und IV b erfolgt hydrogenolytisch z. B. über Palladium auf Kohlenstoff oder mit einem starken Reduktionsmittel, wie z. B. Zinkpulver, Zinn-II-chlorid oder Chrom-II-acetat oder elektrochemisch. Zur Reduktion erwärmt man das Resorufin-Derivat 10 Minuten bis eine Stunde unter einer Wasserstoffatmosphäre oder mit 2 - 10, bevorzugt 2 - 6 Äquivalenten des starken Reduktionsmittels, vorzugsweise Zinn-II-chlorid, in einem geeigneten Lösungsmittel.

Die abschließende Acylierung erfolgt dann in üblicher Weise mit einem Acylierungsmittel der allgemeinen Formel V

X-B (V)

wobei

B die für Verbindungen der allgemeinen Formel I angegebene Bedeutung hat und

X eine reaktive Gruppe

bedeutet.

Als Acylierungsmittel kommen vor allem Säurehalogenide, -anhydride oder -ester in Frage. Besonders bevorzugt werden Säurehalogenide. Vorzugsweise wird das Halogenatom aus der Gruppe Chlor, Brom und Jod ausgewählt. Besonders bevorzugt sind Chlor und Brom.

Bevorzugt werden die Verbindungen der allgemeinen Formel I in einem Eintopf-Verfahren durch reduktive Acylierung der Resorufin-Derivate der allgemeinen Formeln IV a und IV b hergestellt. Das entsprechende Resorufin-Derivat wird hierzu mit zwei bis sechs Äquivalenten des Reduktionsmittels in Gegenwart des Acylierungsmittels (X-B) in einem geeigneten Lösungsmittel fünf Minuten bis drei Stunden unter Rückfluß erhitzt oder bei Raumtemperatur vier bis 16 Stunden gerührt.

Geeignete Lösungsmittel sind dipolare, aprotische Flüssigkeiten oder die dem Acylierungsmittel entsprechenden Säuren. Für die Acetylierung wird z. B. Eisessig als Lösungsmittel eingesetzt.

Zur Herstellung von Verbindungen der allgemeinen Formel I, in der A gleich Z und A ungleich B ist, werden Verbindungen der allgemeinen tautomeren Formeln II a und II b, in denen $R^1$ - $R^6$ und Y die vorgenannte Bedeutung haben, reduziert, mittels einer Verbindung der allgemeinen Formel V

X-B (V)

wobei

B die in der allgemeinen Formel I angegebene Bedeutung hat und

X eine reaktive Gruppe bedeutet,

acyliert, anschließend selektiv die Esterbindungen hydrolisiert und dann mit einer Verbindung der allgemeinen Formel VI

X-A (VI)

wobei A und X die vorgenannte Bedeutung haben, umgesetzt.

Die Reduktion von Resorufin-Derivaten der allgemeinen Formeln II a und II b kann in gleicher Weise erfolgen, wie bereits für die Reduktion von Resorufin-Derivaten der allgemeinen Formeln IV a und IV b beschrieben.

Die Acylierung mittels einer Verbindung der allgemeinen Formel V erfolgt in üblicher Weise. Bevorzugte Acylierungsmittel sind Säurehalogenide, Säureanhydride oder Ester. Bevorzugt eingesetzt werden Säurehalogenide und Säureanhydride. Besonders bevorzugt sind Säurechloride niedriger Alkancarbonsäuren mit 1 - 5 Kohlenstoffatomen. Ganz besonders bevorzugt ist Acetylchlorid.

In einem bevorzugten Verfahren werden die Verbindungen der allgemeinen tautomeren Formeln II a und II b in einem Eintopf-Verfahren reduktiv acyliert. Das entsprechende Resorufin-Derivat wird hierzu unter einer Wasserstoffatmosphäre z. B. über Palladium auf Kohle oder mit 2 - 6 Äquivalenten eines starken Reduktionsmittels in Gegenwart des Acylierungsmittels in einem geeigneten Lösungsmittel fünf Minuten bis drei Stunden unter Rückfluß erhitzt oder bei Raumtemperatur vier bis 16 Stunden gerührt.

Nach Herstellung der peracylierten Dihydroresorufin-Derivate müssen die Beiden O-Acylgruppen selektiv abgespalten werden. Die selektive Spaltung der Resorufinesterbindungen wird durch Reaktion mit zwei bis zehn Mol, bevorzugt zwei bis vier Mol Natriumsulfit in einem Gemisch aus Wasser und einem wasserlöslichen Lösungsmittel, wie 1,4-Dioxan, Methanol oder Ethanol, bevorzugt Wasser/1,4-Dioxan 1:1 erreicht. Die Reaktionstemperatur beträgt 20 - 100° C, bevorzugt 80 - 100° C. Unter diesen Reaktionsbedingungen lassen sich N-Acyl-dihydroresorufine in hohen Ausbeuten herstellen.

Das so hergestellte N-Acyl-dihydroresorufin kann dann mit einer Verbindung der allgemeinen Formel VI (X-A) umgesetzt werden. Diese Reaktion erfolgt grundsätzlich unter den gleichen Bedingungen wie für die Verfahrensvariante a) bereits beschrieben.

Besonders bevorzugt werden nach Verfahrensvariante b) Verbindungen der allgemeinen Formel I hergestellt, in denen die Reste A und Z $PO_3MM'$ bedeuten. Hierzu werden die wie vorstehend beschrieben hergestellten N-Acyl-dihydroresorufin-Derivate mit einem Halogenid des fünfwertigen Phosphors (vorzugsweise in Gegenwart eines anorganischen oder organischen Säurefängers) umgesetzt und die erhaltene Dihalogenphosphonyloxy-Verbindung anschließend hydrolysiert.

Als Halogenide des fünfwertigen Phosphors kommen beispielsweise Phosphoroxidtrichlorid, Phosphorpentachlorid oder Pyrophosphorsäuretetrachlorid in Betracht. Phosphoroxidtrichlorid und Pyrophosphorsäuretetrachlorid werden bevorzugt eingesetzt. Ganz besonders bevorzugt wird Phosphoroxidtrichlorid. Als Säurefänger eignen sich anorganische und organische Basen, beispielsweise Alkalihydroxide, Alkalicarbonate, Trialkylamine oder Pyridin.

Als Lösungsmittel eignen sich die als Flüssigkeit eingesetzten organischen Säurefänger. Es können aber auch als Lösungs- und Verdünnungsmittel andere, vor allem organische Flüssigkeiten, wie Chloroform, Dichlormethan, Acetonitril oder Tetrahydrofuran eingesetzt werden.

Die Reaktion wird vorzugsweise in Gegenwart eines Lösungs- oder Verdünnungsmittels im Temperaturbereich von - 30 bis + 20° C durchgeführt.

Die Hydrolyse der intermediär gebildeten Dichlorphosphonyloxy-Verbindungen geschieht schonend bei Temperaturen von 0 - 20° C durch Neutralisation mit wässriger Alkalihydroxid- oder Alkalicarbonat-Lösung, Erdalkali-Hydroxid-Lösung, Triethylamin oder Triethylammoniumbicarbonat. Die erhaltenen Alkali-, Erdalkali- oder Ammoniumsalze konzentriert man im Vakuum und gewinnt daraus durch geeignete chromatographische Verfahren die gewünschten Diphosphorsäure-dihydroresorufinderivat-Ester. Eine andere Methode

besteht in der Ausfällung der Diphosphorsäureester aus konzentrierter wässriger Alkali-, Erdalkali- oder Ammoniumsalzlösung. Die freien Diphosphorsäureverbindungen können z. B. durch Ansäuern mit Mineralsäure, wie z. B. Salzsäure und anschließende Ausfällung gewonnen werden.

Zur Herstellung von Verbindungen der allgemeinen Formel I, in der A, B und Z alle ungleich sind, kann von Verbindungen der allgemeinen tautomeren Formeln II a und II b ausgegangen werden, in denen $R^1$ - $R^6$ die vorgenannte Bedeutung haben und Y die Gruppe N -> O bedeutet. Nach Umsetzung mit einer Verbindung der allgemeinen Formel VII

X-A′ (VII)

wobei

A′ die in der allgemeinen Formel I für A angegebene Bedeutung hat oder eine übliche Phenol-Schutzgruppe darstellt und

X eine reaktive Gruppe bedeutet,

wird das OH-substituierte Resazurin-Derivat reduziert und mittels einer Verbindung der allgemeinen Formel V

X-B (V)

wobei

B die für Verbindungen der allgemeinen Formel I angegebene Bedeutung hat und

X eine reaktive Gruppe darstellt

vollständing acyliert. Anschließend wird selektiv die OB-Esterbindung hydrolysiert und mit einer Verbindung der allgemeinen Formel III

X-Z (III)

in der

Z die für Verbindungen der allgemeinen Formel I angegebene Bedeutung hat und

X eine reaktive Gruppe darstellt,

umgesetzt. Wenn A′ nicht die in der allgemeinen Formel I für A angegebene Bedeutung hat, wird A′ auf geeignete Weise entfernt. Durch Reaktion mit einer Verbindung der allgemeinen Formel VI

X-A (VI)

in der

A die in der allgemeinen Formel I angegebene Bedeutung hat und

X eine reaktive Gruppe darstellt,

kann dann das Zielmolekül erhalten werden.

Die Reduktion der nach Umsetzung mit X-A′ (VII) erhaltenen Resazurin-Derivate kann in gleicher Weise erfolgen, wie bereits für die Reduktion von Resorufin-Derivaten der allgemeinen Formeln IV a und IV b beschrieben. Es ist jedoch darauf zu achten, daß ein solches Verfahren gewählt wird, daß die OA′-Bindung hiervon nicht berührt wird. Dies ist insbesondere dann wichtig, wenn für A′ Phenol-Schutzgruppen eingesetzt werden, die hydrogenolytisch abgespalten werden können, wie z. B. die Benzylgruppe.

Als Gruppe A′ kann in dem erfindungsgemäßen Verfahren jede Gruppe A verwendet werden, die bis zum Ende der Reaktionsfolge unverändert vorliegt und der letztlich gewünschten Gruppe A entspricht. Es kann jedoch auch eine übliche Phenol-Schutzgruppe A′, wie z. B. die Benzylgruppe, eingesetzt werden, die im Verlauf der Reaktionsfolge durch A ersetzt wird.

Die Umsetzung der nach Reduktion des OH-substituierten Resazurin-Derivats vorliegenden Dihydroresorufinverbindung mit X-B (V) kann so erfolgen, daß selektiv nur die Aminogruppe acyliert wird, so daß anschließend sofort die freie OH-Gruppe mit X-Z (III) umgesetzt werden kann. Bevorzugt werden jedoch beide Gruppen, sowohl die Aminogruppe als auch die freie OH-Gruppe in einem Reaktionsschritt acyliert und anschließend die Esterbindung wieder selektiv hydrolysiert.

Nach Verfahrensvariante c) bei der A, B und Z alle ungleich sind, werden bevorzugt Verbindungen der allgemeinen Formel I hergestellt, in denen der Rest Z einen carboxylgebundenen Aminosäure- oder Oligopeptidrest darstellt, wobei die Aminogruppen oder sonstige funktionelle Gruppen dieser Reste sowohl frei als auch durch übliche Schutzgruppen blockiert vorliegen können. Ganz besonders bevorzugt für die Umsetzung der Verbindung X-Z (III) sind solche Aminosäure- oder Oligopeptidreste, bei denen sämtliche

funktionelle Gruppen geschützt sind. Eine Entfernung dieser Schutzgruppen im Verlauf der Reaktionsfolge zur Herstellung von Verbindungen der allgemeinen Formel I, in der A, Abund Z alle ungleich sind, ist möglich, oft jedoch nicht erforderlich.

Als reaktive Gruppe X in Verbindungen der allgemeinen Formel III kommen in diesem Fall die in der Peptidchemie üblichen Gruppen in Frage.

Wenn bei den nach Verfahrensvariante c) hergestellten Verbindungen Z einen Aminosäure- oder Oligopeptidrest darstellt, hat A bevorzugt die Bedeutung eines $C_1$-$C_5$-Alkylcarbonyl-Restes, der gegebenenfalls durch Carboxyl-, Hydroxyl- und/oder Ammoniumreste ein- oder mehrfach substituiert ist. Ganz besonders bevorzugt sind der Glutarsäure- oder Bernsteinsaurerest.

B stellt bei solchen bevorzugten Verbindungen einen $C_1$-$C_5$-Alkylcarbonylrest dar, besonders bevorzugt einen Acetylrest.

Die bevorzugten reaktiven Gruppen X der Verbindungen der allgemeiner Formeln V und VI sind solche, die üblicherweise zur Aktivierung von Carbonsäuren verwendet werden. Besonders bevorzugt werden Säurehalogenide und Säureanhydride eingesetzt.

Bei Verbindungen der allgemeinen Formel VII, in der A' eine Phenol-Schutzgruppe darstellt, kann die reaktive Gruppe X jede Gruppe sein, die mit phenolischen OH-Gruppen zur Reaktion zu bringen ist. Besonders bevorzugt sind Halogenatome, ganz besonders bevorzugt Chlor und Brom.

Bei der Synthese von Verbindungen der allgemeinen Formel I entstehen gut wasserlösliche, farblose und nicht fluoreszierende Verbindungen. Geeignete Hydrolasen sind in der Lage, die Z-O-Bindung solcher Substrate zu spalten und so, je nachdem, ob Z gleich A oder Z ungleich A ist, freie, nur noch N-acylierte Dihydroresorufine mit zwei freien OH-Gruppen oder N-acylierte Dihydroresorufin-Derivate mit einer freien OH-Gruppe freizusetzen. Solche durch hydrolytische Aktivitäten der Enzyme freigesetzten Verbindungen lassen sich leicht zu farbigen und fluoreszierenden Resorufin-Derivaten oxidieren, die gut detektierbar sind.

Ein weiterer Gegenstand der Erfindung ist somit die Verwendung der Substanzen der allgemeinen Formel I zum Nachweis und zur Bestimmung von Hydrolasen. Je nach Wahl der Reste Z und A sind die erfindungsgemäßen Verbindungen der allgemeinen Formel I grundsätzlich als Substrate für alle möglichen Hydrolasen geeignet. Als besonders vorteilhaft haben sich diese Verbindungen als Substrate für Esterasen und Glycosidasen erwiesen.

Carboxylrester spaltende Esterasen setzen vorzugsweise Verbindungen der allgemeinen Formel I, in der Z einen Alkylcarbonylrest aus 1 - 20 Kohlenstoffatomen oder einen carborylgebundenen Aminosäure- oder Oligopeptidrest und A und B $C_1$-$C_5$-Alkylcarbonyl-Reste, die gegebenenfalls durch Hydroxyl-, Carboxyl- und/oder Ammoniumreste ein- oder mehrfach substituiert sind, darstellen, um. Ein geeignetes Substrat zum Nachweis einer Fettsäureester spaltenden Hydrolase ist z. B. eine Verbindung der allgemeinen Formel I, in der Z einen Propylcarbonylrest und A und B Succinylreste darstellen. Besonders bevorzugt ist N,3-Disuccinyl-7-butyryl-3,7-dihydroxy-phenoxazin.

In Leucozyten vorkommende Carboxylester spaltende Enzyme setzen besonders gut Verbindungen der allgemeinen Formel I um, in denen Z einen Aminosäure- oder Oligopeptidrest bedeutet, z. B. Alanin und A und B gegebenenfalls durch Carboxyl-, Hydroxyl- und/oder Ammoniumreste ein- oder mehrfach substituierte $C_1$-$C_5$-Alkylcarbonylreste, wie z. B. Acetyl-, Carnityl-, Succinyl- oder Glutaryl-Reste darstellen. Besonders geeignet sind N,3-Disuccinyl-7-alanyl-3,7-dihydroxyphenoxazin, N,3-Diglutaryl-7-alanyl-3,7-dihydroxyphenoxazin und N-Acetyl-3-glutaryl-7-alanyl-3,7-dihydroxyphenoxazin. Die Aminogruppe des Aminosäurerestes kann hierbei auch durch übliche Schutzgruppen derivatisiert sein.

Auch saure und alkalische Phosphatasen sind zur Gruppe der Esterasen einzuordnende Enzyme. Verbindungen der allgemeinen Formel I, in der Z $PO_3MM'$ bedeutet und A und B $C_1$-C-Alkylcarbonyl-Reste, die gegebenenfalls durch Hydroxyl-, Carboryl- und/oder Ammoniumreste ein- oder mehrfach substituiert sind, darstellen, können als Substrate für Phosphatasen jeglicher Herkunft eingesetzt werden. Besonders geeignet sind erfindungsgemäße Verbindungen, in denen A und B einen Acetylrest darstellen. Besonders bevorzugt als Phosphatase-Substrat ist N,3-Diacetyl-dihydroresorufin-7-phosphat.

Es sind hierfür jedoch auch Verbindungen der allgemeinen Formel I einsetzbar, in denen A und Z gleichzeitig einen Phosphorsäurerest darstellen. Besonders bevorzugte Verbindungen sind N-Acetyl-dihydroresorufin-3,7-bisphosphat und N-Acetyl-2,8-dibrom-dihydroresorufin-3,7-bisphosphat.

Glycosidase-Substrate sind Verbindungen der allgemeinen Formel I, in der A und B gleich sind, $C_1$-$C_5$-Alkylcarbonyl-Reste bedeuten, die gegebenenfalls durch Hydroxyl-, Carboxyl- und/oder Ammoniumreste ein- oder mehrfach substituiert sind und Z einen Zuckerrest darstellt. Als Zuckerrest kommt prinzipiell jedes Mono- oder Oligosaccharid in Frage, welches durch eine entsprechende Glycosidase von dem Dihydroresorufin-Grundgerüst abgespalten werden kann. Bevorzugte Monosaccharide sind $\alpha$- oder $\beta$-glycosidisch gebundene Glucose, Galactose oder Mannose. Substrate, die zum Nachweis von Glucosidase geeignet sind, sind z. B. N,3-Diacetyl-dihydroresorufin-7-glucosid, N,3-Disuccinyl-dihydroresorufin-7-glucosid oder

N,3-Diglutaryl-dihydroresorufin-7-glucosid. Ein bevorzugtes Galatosidase-Substrat ist z. B. N,3-Diacetyl-dihydroresorufin-7-galactosid.

Die nach enzymatischer Hydrolyse durch Oxidation und Hydrolyse entstehenden Produkte der N,3-Diacyl-dihydroresorufin-Derivate sind außer für UV-photometrische und visuelle Detektion vor allem auch für fluorimetrische Messungen geeignet. Dies ist deshalb von Bedeutung, weil gegenüber photometrischen Methoden die Empfindlichkeit fluorimetrischer Bestimmungen oft um einige Zehnerpotenzen erhöht ist. In manchen Fällen muß mit fluorogenen Substraten gearbeitet werden, beispielsweise bei der Untersuchung enzymatischer Aktivitäten in Zellen mit automatischen Geräten zur Zelldifferenzierung (Zytofluorometrie) sowie bei der Analyse immobilisierter Enzyme, wie Durchflußmikrofluorometrie. In anderen Fällen, z. B. bei der Bestimmung der enzymatischen Markierung von Testsystem (Enzymimmunoassays), die z. B. oft mit β-Galactosidase, aber auch mit anderen Hydrolasen durchgeführt wird, wird der Multiplikationseffekt der enzymatischen Katalyse durch Verwendung fluorogener Substrate beträchtlich verstärkt.

Als besonders vorteilhaft zur Bestimmung von Hydrolasen hat sich der große Unterschied der maximalen Absorptionswellenlängen von Substrat und resultierendem, oxidiertem Produkt erwiesen. Alle Verbindungen der allgemeinen Formel I sind farblose Substanzen. Die enzymatisch hydrolysierten und oxidierten Verbindungen sind dagegen rot bis blaurot gefärbt.

Besonders hervorzuheben ist außerdem die gute Wasserlöslichkeit der beanspruchten Verbindungen der allgemeinen Formel I. Damit entfällt die Notwendigkeit des Zusatzes organischer Lösungsmittel oder von Detergentien zu enzymatischen Tests, um das verwendete Chromogen in Lösung zu bringen. Dieser Vorteil wirkt sich unter anderem bei kinetischen Enzymtests aus, wo gute Löslichkeiten von Substrat und Produkt erforderlich sind und wo Lösungsmittel- oder Detergenzzusätze oft die Enzymaktivitäten beeinflussen.

Zur Bestimmung von Hydrolasen werden eine Verbindung der allgemeinen Formel I, ein Oxidationsmittel, ein geeignetes Puffersystem sowie gegebenenfalls weitere Reagenzien und Hilfsstoffe mit der Probe, die das zu bestimmende Enzym enthält, vermischt. Die N-Acyl-dihydroresorufin-Derivate der allgemeinen Formel I werden bei Anwesenheit einer entsprechenden Hydrolase zu einem Dihydroresorufin-Derivat mit mindestens einer freien OH-Gruppe umgesetzt. Dieses wird durch das anwesende Oxidationsmittel oxidiert. Die dadurch bewirkte Änderung der Absorption bzw. Fluoreszenzintensität der Reaktionsmischung wird photometrisch bzw. fluorimetrisch gemessen. Durch direkten Vergleich mit einer Standardlösung oder durch indirekten Vergleich mit einer Standardkurve kann so der Gehalt an zu bestimmendem Enzym in der Probe ermittelt werden. Es sind sowohl kinetische als Endpunkts-Messungen möglich.

Für die Oxidation des durch Hydrolase-Einwirkung zunächst entstehenden Dihydroresorufins mit mindestens einer freien OH-Gruppe kann jedes Oxidationsmittel verwendet werden, welches die Aktivität des zu bestimmenden Enzyms nicht beeinflußt und welches stark genug ist, das resultierende Dihydroresorufin zu oxidieren. Je nach den Substituenten kann hierzu bereits Luftsauerstoff genügen. In der Regel werden als Oxidationsmittel Kaliumhexacyanoferrat-(III), Perborat, Bilirubinoxidase oder Peroxidase/Wasserstoffperoxid eingesetzt.

Das Reagenz zur Bestimmung von Hydrolasen enthält neben einem oder mehreren der erfindungsgemäßen Substrate der allgemeinen Formel I, ein Oxidationsmittel, ein geeignetes Puffersystem sowie gegebenenfalls weitere geeignete, üblicherweise für solche Reagenzien verwendete Zusatzstoffe, wie beispielsweise weitere Hilfsenzyme, Stabilisatoren usw. Das erfindungsgemäße Reagenz kann in Form einer Lösung, als Lyophilisat, als Pulvergemisch, Reagenztablette oder ein geeignetes Trägermaterial aufgebracht vorliegen.

Das erfindungsgemäße Reagenz in Form einer Lösung enthält vorzugsweise sämtliche für den Test benötigten Reagenzien. Als Lösungsmittel kommen Wasser oder Gemische von Wasser mit einem wasserlöslichen organischen Lösunsmittel, wie z. B. Methanol, Ethanol, Aceton oder Dimethylformamid in Frage. Aus Haltbarkeitsgründen kann es vorteilhaft sein, die für den Test benötigten Reagenzien auf zwei oder mehr Lösungen zu verteilen, die erst bei der eigentlichen Untersuchung vermischt werden.

Zur Herstellung des Reagenzes in Form eines Lyophilisats im Gesamtgewicht von jeweils etwa 5 - 20 mg, vorzugsweise etwa 10 mg, wird eine Lösung getrocknet, die neben sämtlichen für den Test benötigten Reagenzien übliche Gerüstbildner, wie z. B. Polyvinylpyrrolidon und eventuelle weitere Füllstoffe, wie z. B. Mannit, Sorbit oder Xylit enthält.

Ein Reagenz in Form von Pulvermischungen oder Reagenztabletten läßt sich herstellen, indem die Bestandteile des Testes mit üblichen galenischen Zusatzstoffen versetzt und granuliert werden. Zusatzstoffe dieser Art sind z. B. Zuckeralkohole, wie Mannit, Sorbit oder Xylit, oder andere lösliche inerte Verbindungen, wie Polyethylenglykole oder Polyvinylpyrrolidon. Die Pulvermischungen oder Reagenztabletten weisen im allgemeinen ein Endgewicht von ungefähr 3 - 200 mg, vorzugsweise 50 - 80 mg, auf.

Zur Herstellung des Reagenzes in Form eines Teststreifens wird ein saugfähiger Träger, vorzugsweise Filterpapier, Zellulose oder Kunstfaservlies, mit Lösungen der erforderlichen, üblicherweise zur Herstellung von Teststreifen verwendeten Reagenzien in leicht flüchtigen Lösungsmitteln, wie z. B. Wasser, Methanol, Ethanol oder Aceton, imprägniert. Dies kan in einem Imprägnierschritt erfolgen. Oft ist es jedoch zweckmäßig, die Imprägnierung in mehreren Schritten durchzuführen, wobei Lösungen eingesetzt werden, die jeweils einen Teil der Bestandteile des Reagenzes enthalten. So kann beispielsweise in einem ersten Schritt mit einer wässrigen Lösung, die das Oxidationsmittel, den Puffer und andere wasserlösliche Zusatzstoffe enthält, und dann in einem zweiten Schritt mit einer Lösung, die das Hydrolase-Substrat enthält, imprägniert werden. Die fertigen Teststreifen können als solche verwendet werden oder in an sich bekannter Weise an Griffen angeklebt oder vorzugsweise zwischen Kunststoffen und feinmaschigen Netzwerken gemäß DBP 21 18 455 eingesiegelt werden.

Die nachfolgenden Beispiele zeigen einige der zahlreichen Verfahrensvarianten, die zur Synthese der erfindungsgemäßen Verbindung beschritten werden können sowie beispielhaft die Verwendung der neuen Hydrolase-Substrate zur Bestimmung der Aktivität von entsprechenden Hydrolasen.

## Beispiel 1

### N-Acetyl-dihydroresorufin-3,7-bisphosphat-tetranatriumsalz

#### Triacetyl-3,7-dihydroxyphenoxazin

2,51 g (10 mmol) Resazurin-Natriumsalz und 4,17 g (22 mmol) Zinn-II-chlorid wurden in einem Gemisch aus 6,7 ml (72 mmol) Essigsäureanhydrid und 8 ml Eisessig eine Stunde unter Rückfluß erwärmt, wobei allmählich eine klare, braune Lösung entstand. Im Eisbad wurde dann unter Rühren mit ca. 30 ml Methanol versetzt, wobei spontan Kristallisation eintrat. Nach 20 Stunden bei 0 °C wurde abgesaugt, mit eiskaltem Isopropanol und Petrolether gewaschen und am Vakuum über Calciumchlorid getrocknet.
Ausbeute: 2,64 g (77 % d. Th.) beigefarbene Nadeln
Schmelzpunkt: 208 °C
Rf-Wert: 0,45 (DC auf Kieselgel, Chloroform/Essigester = 2/1)

#### N-Acetyl-3,7-dihydroxyphenoxazin

2,39 g (7 mmol) Triacetyl-3,7-dihydroxyphenoxazin und 1,9 g (15 mmol) Natriumsulfit wurden in 70 ml mit Stickstoff begastem Dioxan-Wasser-Gemisch(1:1) gelöst und 20 Minuten unter Rückfluß erwärmt. Es wurde im Eisbad abgekühlt, mit 1,14 ml (20 mmol) Essigsäure angesäuert, von wenig ungelöstem über Watte abfiltriert und auf ca. 20 ml eingeengt. Hierbei sonderte sich ein zähes Öl ab. Ca. 150 ml Stickstoff begastes Wasser wurden zugegeben und die Mischung bei 0 °C stehengelassen. Die ausfallenden Kristalle wurden abgesaugt, mit Eiswasser gewaschen und am Vakuum bei 0 °C über Calciumchlorid getrocknet.
Ausbeute: 1,5 g (97 % d. Th.) beigefarbenes Produkt
Schmelzpunkt: > 250° (Zers.)
Rf-Wert: 0,19 (DC auf Kieselgel, Chloroform/Essigester = 2/1)

#### N-Acetyl-dihydroresorufin-3,7-bisphosphat-tetranatriumsalz

Ein Gemisch aus 1,29 g (5 mmol) N-Acetyl-dihydroresorufin und 1,62 ml (20 mmol) Phosphoroxidtrichlorid in 5 ml absolutem Tetrahydrofuran wird bei 0 °C unter Rühren innerhalb 20 Minuten in 10 ml absolutes Pyridin getropft. Man entfernt das Eisbad und läßt bei Raumtemperatur weitere zwei Stunden rühren. Die Reaktionsmischung wird dann auf 50 g Eis gegossen und nach drei Stunden auf ca. 20 ml eingeengt, mit 2 N wässriger Natronlauge auf pH 8 eingestellt und über Nacht 0 °C auskristallisiert. Man saugt ab, wäscht mit wenig Eiswasser, Isopropanol und Petroläther und trocknet bis zur Gewichtskonstanz.
Ausbeute: 1,5 g (59 %) farbloses Pulver
Rf-Wert: 0,71 (DC auf Zellulose, 1 M wässrige Ammoniumacetat-Lösung/Ethanol = 5/2)

Beispiel 2

N-Acetyl-2,8-dibrom-dihydroresorufin-3,7-bisphosphat-tetralithiumsalz

Triacetyl-2,8-dibrom-dihydroresorufin

7,4 g (20 mmol) 2,8-dibromresorufin (AFANASIEVA, GB et al, Khim. Geterotsek L. Soedin (1974), Seite 348 - 353) werden in einem Gemisch aus 150 ml Eisessig und 50 ml Acetanhydrid unter Zugabe von 0,8 g Palladium auf Kohle (10 %) bis zur Aufnahme der theoretischen Menge Wasserstoff katalytisch hydriert. Man spült kräftig mit sauerstoffreiem Stickstoff und erhizt eine Stunde unter Feuchtigkeitsausschluß am Rückfluß. Man filtriert heiß und engt im Vakuum zur Trockne ein.
Ausbeute: 3,5 g farblose Kristalle

N-Acetyl-2,8-dibrom-dihydroresorufin

3,5 g (7 mmol) Triacetyl-2,8-dibrom-dihydroresorufin werden in 30 ml Dioxan aufgenommen, mit einer Lösung von 0,95 g Natriumsulfit (7,5 mmol) in 30 ml Wasser versetzt und 20 Minuten unter Rückfluß erwärmt. Man engt im Vakuum zur Trockne ein und homogenisiert mit ca. 80 ml Eiswasser. Der Niederschlag wird abgesaugt, mit Eiswasser gewaschen und getrocknet.
Ausbeute: 2,15 g (74 %) leicht graues Pulver
Schmelzpunkt: 189 - 190° C

N-Acetyl-2,8-dibrom-dihydroresorufin-3,7-bisphosphat-tetralithiumsalz

2 g (4,8 mmol) N-Acetyl-2,8-dibrom-dihydroresorufin werden in 50 ml absolutem Pyridin gelöst und innerhalb zwei Stunden bei 0° C in ein Gemisch aus 1,77 ml (19,3 mmol) Phosphoroxidtrichlorid und 30 ml absolutes Pyridin getropft. Man läßt auf Raumtemperatur erwärmen und rührt eine weitere Stunde. Das Gemisch wird auf 100 g Eis gegossen, mit 2 N wässriger Lithiumhydroxidlösung auf pH 8 gestellt, von Lithiumphosphat abgesaugt und im Vakuum auf 50 ml eingeengt.

Man chromatographiert über 1,7 l Sephadex LH 20 (50 % wässriges Methanol und fraktioniert in 10 ml-Portionen. Die Phosphatase-positiven Fraktionen werden vereinigt und im Vakuum zur Trockne eingeengt. Der Rückstand wird in 200 ml Aceton digeriert, abgesaugt und getrocknet.
Ausbeute: 2,75 g (95 %)
Rf-Wert: 0.57 (DC auf Zellulose. 1 M wässrige Ammoniumacetat-Lösung/Ethanol = 5/2)

Beispiel 3

Bestimmung der Aktivität von saurer Phosphatase

N-Acetyl-2,8-dibrom-dihydroresorufin-3,7-bisphosphat-tetralithiumsalz wird in einer Konzentration von 1 mmol/l in 0,1 M Citrat-Puffer, pH 5,2, enthaltend 0,1 mmol Kaliumhexacyanoferrat-III/l, gelöst.

Zu 2,00 ml dieser Lösung werden in einer Küvette 0,20 ml Serum enthaltend saure Phosphatase gegeben. Nach Mischung wirde fünf Minuten bei 30° C inkubiert, dann die Anfangsextinktion bei einer Meßwellenlänge von 578 nm abgelesen und diese Ablesung nach genau 1, 2 und 3 Minuten wiederholt. Aus den Extinktionsdifferenzen pro Minute (ΔE/min) wird ein Mittelwert gebildet und dieser in die Gleichung zur Berechnung der Enzymaktivität eingesetzt.

Die Aktivität der sauren Phosphatase berechnet sich nach:

$$U/l = \frac{1000 \times V}{\varepsilon \times d \times v} \times \Delta E/min$$

U/l = Aktivität des Enzyms in Einheiten pro Liter
V = Gesamtvolumen in ml
v = Probevolumen in ml
$\epsilon$ = Extinktionskoeffizient der Indikatorsubstanz in l . $mmol^1$ . $cm^{-1}$

d = Schichtdicke der Küvette in cm

Beispiel 4

Bestimmung von alkalischer Phosphatase

N-Acetyldihydroresorufin-3,7-bisphosphat-tetranatriumsalz wird in einer Konzentration von 1 mmol/l in 0,1 M Diethanolamin-Puffer, pH 9,8, enthaltend 0,1 mmol Kaliumhexacyanoferrat-III/l, gelöst. Bei Zugabe einer Lösung, die alkalische Phosphatase enthält, erfolgt ein Farbumschlag von farblos nach blaurot.

Die Intensität der Verfärbung ist der Konzentration der alkalischen Phosphatase in der Probe proportional.

Mit Hilfe von Proben mit bekannter Konzentration an alkalischer Phosphatase läßt sich eine Eichkurve ermitteln, anhand derer der unbekannte Phosphatase-Gehalt einer Probe bestimmt werden kann.

Beispiel 5

7,10-Diacetyl-3,7-dihydroxyphenoxazin-3-phosphat-lithiumsalz

O-Acetyl-resorufin

Ein Gemisch von 21,3 g (0.1 mol) Resorufin, 50 ml Acetanhydrid und 25 ml Eisessig werden vier Stunden unter Rückfluß erhitzt. Man läßt über Nacht bei 0 °C stehen, rührt den entstandenen Kristallbrei mit Ether/Petrolether (1:1) auf und saugt die Kristalle ab. Die Kristalle werden mit Petrolether nachgewaschen und am Vakuum bei 60 °C über Calciumchlorid getrocknet.
Ausbeute: 18,5 g (72,5 %) orangefarbenes Kristallisat
Schmelzpunkt: 185 - 190 °C

N,O-Diacetyl-dihydroresorufin

12,8 g (50 mmol) O-Acetyl-resorufin, 5,7 ml (60 mmol) Acetanhydrid und 0,6 g Pd/C werden in 120 ml Eisessig 6 Stunden lang hydriert. Das Gemisch wird mit Wasser verdünnt, 5 Stunden bei 0 °C stehengelassen und die Kristalle dann abgesaugt. Das Kristallisat wird in Aceton gelöst und vom Katalysator abfiltriert. Die Lösung wird mit Wasser versetzt, eingeengt und 20 Stunden bei 0 °C stehengelassen. Die gebildeten Kristalle werden abgesaugt, mit Wasser gewaschen und am Vakuum bei 50 °C über Calciumchlorid getrocknet.
Ausbeute: 14,6 g (97,5 %) Kristallisat
Schmelzpunkt: 182 °C

7,10-diacetyl-3,7-dihydroxyphenoxazin-3-phosphat-lithiumsalz

1,5 g (5 mmol) N,0-Diacetyldihydroresorufin werden in ein Gemisch von 1,53 g (0,92 ml, 10 mmol) Phosphoroxychlorid, 5,6 ml (70 mmol) absolutem Pyridin und 20 ml absolutem Dioxan eingetragen und 20 Stunden bei Raumtemperatur gerührt. Das Gemisch wird auf 50 g Eis gegossen, die wässrige Lösung auf 240 ml DEAE-Sephadex$^R$ aufgezogen und mit einer 0,5 n wässrigen Lithiumchlorid-Lösung fraktioniert chromatographiert. Die vereinigten Produktfraktionen werden eingeengt, zweimal mit je 200 ml Aceton versetzt, homogenisiert und jeweils dekantiert, zuletzt abgesaugt, mit Aceton nachgewaschen und am Vakuum über Calciumchlorid getrocknet. Das Produkt wird auf 1,2 l Diaion$^R$ mit Wasser aufgezogen und unter Zusatz von Isopropanol eluiert.
Ausbeute: 1,42 g (74 %) hellgraues Pulver

Beispiel 6

N,O-Diacetyl-dihydroresorufin-D-galactopyranosid

N,O-Diacetyl-dihydroresorufin 2,3,4,6-tetra-O-benzyl-D-galactopyranosid

Eine Lösung aus 6,8 g (10,0 mmol) O-(2,3,4,6-Tetra-O-benzyl-α-D-galactopyranosyl)trichloracetimidat (Synthese gemäß Angewandte Chemie 92, 763 (1980), 1,5 g (5,0 mmol) N,O-Diacetyldihydroresorufin und

1,7 g (12,0 mmol) Bortrifluoridetherat in 30 ml wasserfreiem Dichlormethan wird sechs Stunden unter Rückfluß gerührt. Man läßt auf 20 °C abkühlen und schüttelt die Reaktionslösung mit 100 ml einer 1 M Natriumhydrogencarbonat-Lösung aus. Die organische Phase wird über Natriumsulfat getrocknet und im Vakuum eingedampft. Das so erhaltene Produkt wird durch Säulenchromatographie an Kieselgel (Flashchromatographie, Eluens Chloroform:Petrolether = 8:2) gereinigt.
Ausbeute (Anomerengemisch): 1,5 g (36 % bezogen auf N,O-Diacetyldihydroresorufin)
$R_F$ (Kieselgel, Chloroform: Ethylacetat = 9:1) = 0,63
MS: m/e = 821

N,O-Diacetyl-dihydroresorufin-D-galactopyranosid

Zur Abspaltung der Benzylschutzgruppen werden 1,5 g (1,8 mmol) N,O-Diacetyl-dihydroresorufin 2,3,4,6-tetra-O-benzyl-D-galactopyranosid in 70 ml Dichlormethan gelöst und nach Zugabe von 3 g Pd-Mohr unter Normaldruck hydriert. Die Reaktion ist nach etwa zwei Stunden beendet. Nach Abtrennung des Katalysators wird die Reaktionslösung im Vakuum eingeengt, das so erhaltene Rohprodukt durch Säulenchromatographie an Kieselgel (Flashchromatographie, Eluens Chloroform:Ethylacetat = 9:1) aufgereinigt und das Lösungsmittel im Vakuum entfernt.
Ausbeute: 100 mg (12 %)
$R_F$ (Kieselgel, Chloroform:Methanol = 8:2) = 0,33
MS (pos. FAB): m/e = 462, MS (neg. FAB): m/e = 460

Beispiel 7

7,10-Disuccinyl-3,7-dihydroxyphenoxazin-3-butyrat

Resorufin-O-butyrat

2,13 g (10 mmol) Resorufin werden in einem Gemisch von 8 ml (50 mmol) Buttersäureanhydrid und 5 ml absolutem Dimethylformamid gelöst und 20 Stunden bei 50 °C unter Feuchtigkeitsausschluß gerührt. Das Gemisch wird dann am Vakuum eingeengt und mit Petrolether versetzt, wobei Kristallisation eintritt. Die Kristalle werden aus Aceton/Wasser unkristallisiert.
Ausbeute: 3,15 g (85 %) organgefarbene Nadeln
Schmelzpunkt 133 - 134 °C

7,10-Disuccinyl-3,7-dihydroxyphenoxazin-3-butyrat

3 g Resorufin-O-butyrat werden in 50 ml absolutem Tetrahydrofuran gelöst und mit 1,8 g Bernsteinsäureanhydrid, 1,7 g Zinn(II)chlorid, 0,5 ml Eisessig sowie einer Spatelspitze 4-(N,N-Dimethylamino)pyridin versetzt. Man rührt 12 Stunden bei 80 °C und engt zur Trockne ein. Der Rückstand wird aus Ethanol kristallisiert.
Ausbeute: 2 g farbloses Kristallisat
$R_F$ (Kieselgel, Chloroform : Ethylacetat = 2:1) =0,45

Beispiel 8

O-(N′-Tos-Ala)-N-acetyl-O-glutaryl-dihydroresorufin

O-Benzylresazurin

Eine Lösung aus 25,1 g (0,1 mol) Resazurin-Natriumsalz, 8,4 g (0,1 mol) Natriumhydrogencarbonat, 1,6 g (0,01 mol) Kaliumjodid und 14,3 ml (0,12 mol) Benzylbromid in 300 ml Dimethylformamid wird 22 Stunden bei 100 °C gerührt. Nach Erkalten gießt man die Reaktionslösung in 2 l Eiswasser und filtriert nach 2 Stunden das ausgefallene Produkt ab. Anschließend wird mit Eiswasser nachgewaschen, bis das Filtrat farblos ist. Das Produkt trocknet man bei 35 °C über Calciumchlorid im Vakuum.
Ausbeute: 20,5 g (64 %)
Schmp.: 205 - 207 °C (Zersetzung)
$R_F$ (Kieselgel, Chloroform : Aceton = 7:3) = 0,75

N,O-Diacetyl-O-benzyl-dihydroresorufin

10 g (31 mmol) O-Benzylresazurin werden in 250 ml Eisessig gelöst und langsam unter Rühren mit 22 g (336 mmol) Zinkpulver versetzt und das Reaktionsgemisch 1 Stunde unter Rückfluß gekocht. Nach dem Erkalten werden langsam 100 ml Acetanhydrid zugetropft und erneut 5 Stunden unter Rückfluß gekocht. Man läßt das Reaktionsgemisch abkühlen, filtriert vom Ungelösten ab und dampft das Filtrat im Vakuum zur Trockne ein. Der Rückstand wird mit Ethylacetat aufgenommen, mit 5 %iger Natriumhydrogencarbonat-Lösung und Wasser ausgeschüttelt und die organische Phase über Natriumsulfat getrocknet. Das nach dem Eindampfen der Lösung erhaltene Rohprodukt wird aus Toluol umkristallisiert.
Ausbeute: 6,4 g (53 %)
Schmp.: 134 - 136 °C
$R_F$ (Kieselgel, Chloroform : Aceton = 7:3) = 0,87

N-Acetyl-O-benzyl-dihydroresorufin

6,2 g (16 mmol) N,O-Diacetyl-O-benzyl-dihydroresorufin werden in 150 ml Dioxan gelöst und unter Rühren eine Lösung von 2,25 g (17,5 mmol) Natriumsulfit in 150 ml Wasser bei 20 °C zugegeben. Das Reaktionsgemisch erhitzt man eine Stunde auf 90 °C. Nach dem Abkühlen schüttelt man mehrfach mit Ethylacetat aus, trocknet die organische Phase über Natriumsulfat und dampft das Lösungsmittel im Vakuum ab. Die Aufreinigung erfolgt durch Säulenchromatographie an Kieselgel (Laufmittel Chloroform : Aceton = 7:3).
Ausbeute: 4,3 g (78 %)
Schmp.: 170 - 180 °C (Oxidation)
$R_F$ (Kieselgel, Chloroform: Aceton = 7:3) = 0,79

O-(N′-Tos-Ala)-N-acetyl-O-benzyl-dihydroresorufin

3,47 g (10 mmol) N-Acetyl-O-benzyl-dihydroresorufin werden in 10 ml absolutem Tetrahydrofuran gelöst und bei 0 °C unter Feuchtigkeitsausschluß und Rühren nacheinander mit 3,93 g (15 mmol) des Toluolsulfonylgeschützten Säurechlorids von Alanin und 2,1 ml (15 mmol) Triethylamin versetzt und 18 Stunden bei Raumtemperatur gerührt. Das entstandene Triethylaminhydrochlorid wird abgesaugt und die Lösung am Vakuum bis zur Trockne eingeengt, wobei das Produkt als Harz anfällt.
Rf = 0,51, Chloroform/Ethylacetat (2:1)

O-(N′-Tos-Ala)-N-acetyl-O-glutaryl-dihydroresorufin

5,9 g O-(N′-Tos-Ala)-N-acetyl-O-benzyl-dihydroresorufin werden in 20 ml Tetrahydrofuran gelöst und mit 0,6 g Pd/C versetzt. Man erhitzt zum Sieden und leitet über sechs Stunden einen konstanten Strom von Wasserstoff ein. Die abgekühlte Lösung wird filtriert, mit 1,4 g Glutarsäureanhydrid und einer Spatelspitze 4-(N,N-Dimethylamino)-pyridin versetzt und weitere 18 Stunden zum Sieden erhitzt. Man engt dann zur Trockne ein. Die Aufreinigung erfolgt durch Säulenchromatographie an Kieselgel (Laufmittel Chloroform:Ethylacetat = 3:1).
Ausbeute: 3,5 g farbloses Kristallisat
$R_F$ (Kieselgel, Chloroform : Ethylacetat = 2:1) = 0,27

## Patentansprüche

**1.** Farblose und wasserlösliche Verbindungen der allgemeinen Formel I

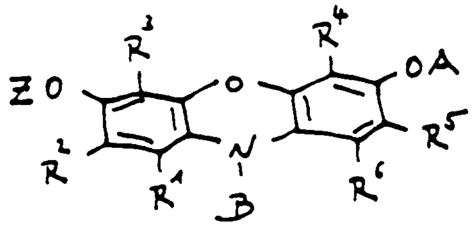

(I)

in der

| | |
|---|---|
| Z | einen organischen oder anorganischen Säurerest oder einen Zuckerrest, |
| A | einen organischen oder anorganischen Säurerest, |
| B | einen organischen Säurerest und |
| $R^2$ und $R^5$, | die gleich oder verschieden sein können, Wasserstoff, Halogen oder eine $C_1$-$C_5$-Alkylgruppe, |
| $R^1$, $R^3$, $R^4$ und $R^6$, | die gleich oder verschieden sein können, Wasserstoff, Halogen, eine Cyano-, $C_1$-$C_5$-Alkyl-, $C_1$-$C_5$-Alkoxy-, Carboxy-, $C_1$-$C_5$-Alkoxycarbonyl-, Carboxy-$C_1$-$C_5$-alkyl-, $C_1$-$C_5$-Alkoxycarbonyl- $C_1$-$C_5$-alkyl- oder eine gegebenenfalls ein oder zweifach substituierte carboxamidogruppe oder die Gruppe |

$-COO-(CH_2CH_2O)_n-R^7$

in der

| | |
|---|---|
| $R^7$ | Wasserstoff oder eine $C_1$-$C_5$-Alkyl-Gruppe und n eine ganze Zahl von 1 bis 4 bedeuten, wobei |
| $R^6$ | zusätzlich eine Sulfo- oder Nitrogruppe vorstellen kann, und wobei |

A, B und Z nicht gleichzeitig eine identische Acylgruppe sind, bedeuten.

**2.** Verbindungen gemäß Anspruch 1,dadurch gekennzeichnet, daß

| | |
|---|---|
| Z | $PO_3MM'$, $SO_3M$, einen carboxylgebundenen Alkancarbonsäure-, Aminosäure- oder Oligopeptidrest, einen Zuckerrest, |
| A | $PO_3MM'$, $SO_3M$, einen $C_1$-$C_5$-Alkylcarbonyl-Rest,der gegebenenfalls durch Carboxyl-, Hydroxyl- und/oder Ammoniumreste ein- oder mehrfach substituiert ist oder einen carboxylgebundenen Aminosäure- oder Oligopeptidrest, |
| M und M' | Wasserstoff, ein Alkali-, Erdalkali- oder Ammoniumion und |
| B | einen gegebenenfalls durch Carboxyl-, Hydroxyl- und/oder Ammoniumreste ein- oder mehrfach substituierten $C_1$-$C_7$-Alkylcarbonyl-Rest |

bedeuten sowie
$R^1$-$R^7$ die oben angegebene Bedeutung haben.

**3.** Verfahren zur Herstellung von farblosen und wasserlöslichen Verbindungen nach Anspruch 1, dadurch gekennzeichnet, daß man in an sich bekannter Weise dann, wenn

a) A ungleich Z und A gleich B
Verbindungen der allgemeinen tautomeren Formeln II a und II b

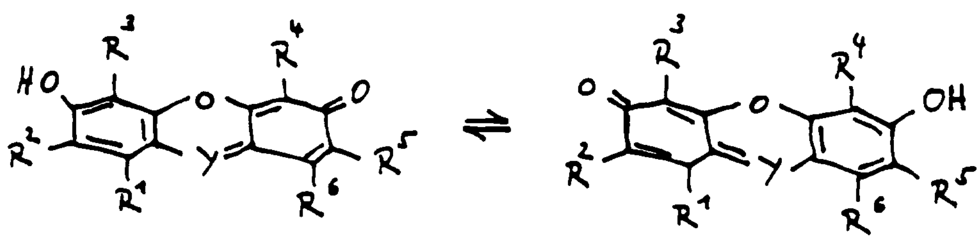

(II a) (II b)

in denen
$R^1$-$R^6$ die oben angegebene Bedeutung haben und Y Stickstoff oder die Gruppe N → O bedeuten, mit

X-Z (III)

wobei

| | |
|---|---|
| Z | die oben angegebene Bedeutung hat und |
| X | eine reaktive Gruppe bedeutet, umsetzt und das als Zwischenprodukt entstehende Resorufin-Derivat der tautomeren Formeln IV a und IV b |

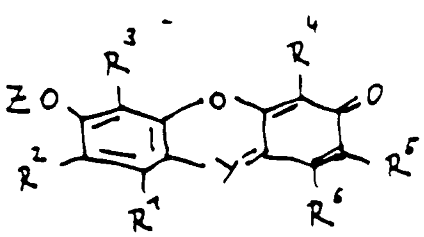

(IV a)

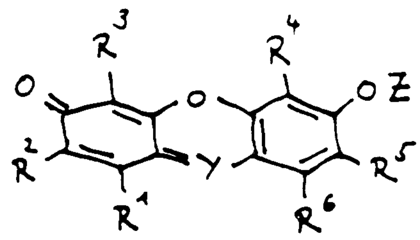

(IV b)

in denen
$R^1$-$R^6$, Y und Z die oben angegebene Bedeutung haben,
reduziert und mittels

X-B (V)

wobei
B die oben angegebene Bedeutung hat und
X eine reaktive Gruppe bedeutet, acyliert,
wenn
b) A gleich Z und A ungleich B
Verbindungen der allgemeinen tautomeren Formeln II a und II b
in denen
$R^1$ - $R^6$ und Y die oben angegebene Bedeutung haben
reduziert und mittels

X-B (V)

wobei
B die oben angegebene Bedeutung hat und
X eine reaktive Gruppe bedeutet
acyliert, anschließend selektiv die Esterbindungen hydrolysiert und dann mit

X-A (VI)

wobei
A und X die oben angegebene Bedeutung haben, umsetzt
oder wenn
c) A, B und Z alle ungleich sind,
Verbindungen der allgemeinen tautomeren Formeln II a und II b
in denen
$R^1$ - $R^6$ die oben angegebene Bedeutung haben und
Y die Gruppe N -> O darstellt,
mit

X-A' (VII)

wobei
A' die oben für A angegebene Bedeutung hat oder eine Phenol-Schutzgruppe darstellt und
X eine reaktive Gruppe bedeutet,
umsetzt, anschließend reduziert und mit

X-B (V)

wobei
B die oben angegebene Bedeutung hat und

X eine reaktive Gruppe darstellt,
erschöpfend acyliert, selektiv die zuletzt gebildete Esterbindung hydrolysiert, mit

X-Z (III)

wobei
Z die oben angegebene Bedeutung hat und
X eine reaktive Gruppe darstellt,
umsetzt und gegebenenfalls, dann wenn A'eine Phenol-Schutzgruppe darstellt, diese entfernt und mit

X-A (VI)

wobei
A die oben angegebene Bedeutung hat und
X eine reaktive Gruppe darstellt,
acyliert.

**4.** Verwendung von Verbindungen gemäß Anspruch 1 zur Bestimmung von Hydrolasen.

**5.** Reagenz zur Bestimmung von Hydrolasen enthaltend ein oder mehrere chromogene oder/und fluorogene Substrate, ein geeignetes Puffersystem sowie gegebenenfalls weitere üblicherweise verwendete Reagenzien und Hilfsstoffe, dadurch gekennzeichnet, daß es als chromogene oder/und fluorogene Substrate Verbindungen gemäß Anspruch 1 oder 2 sowie ein Oxidationsmittel enthält.

**6.** Reagenz gemaß Anspruch 6 , dadurch gekennzeichnet, daß es als Oxidationsmittel $K_3[Fe(CN)_6]$, Perborat, Luftsauerstoff, Bilirubinoxidase oder Wasserstoffperoxid/Peroxidase enthält.

## Claims

**1.** Colourless and water-soluble compounds of the general formula I

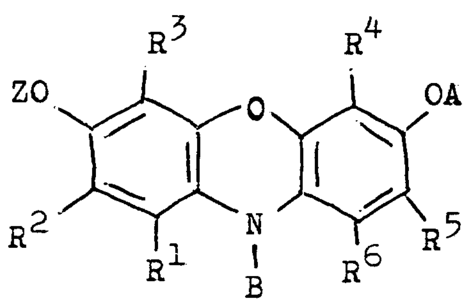

(I)

in which Z signifies an organic or inorganic acid residue or a sugar residue, A an inorganic or organic acid residue, B an organic acid residue, $R^2$ and $R^5$, which can be the same or different, hydrogen, halogen or a $C_1$-$C_5$-alkyl group, $R^1$, $R^3$, $R^4$ and $R^6$, which can be the same or different, hydrogen, halogen, a cyano, $C_1$-$C_5$-alkyl, $C_1$-$C_5$-alkoxy, carboxyl, $C_1$-$C_5$-alkoxycarbonyl, carboxy-$C_1$-$C_5$-alkyl, $C_1$-$C_5$-alkoxycarbonyl-$C_1$-$C_5$-alkyl or a carboxamido group possibly substituted once or twice or the group

$-COO-(CH_2CH_2O)_n-R^7$

in which $R^7$ signifies hydrogen or a $C_1$-$C_5$-alkyl group and n a whole number from 1 to 4, whereby $R^6$ can additionally represent a sulpho or nitro group and whereby A, B and Z do not simultaneously signify an identical acyl group.

**2.** Compounds according to claim 1, characterised in that Z signifies $PO_3MM'$, $SO_3M$, a carboxyl-bound alkanecarboxylic, amino acid or oligopeptide residue, a sugar residue, A $PO_3MM'$, $SO_3M$, a $C_1$-$C_5$-

alkylcarbonyl radical, which is possibly substituted one or more times by carboxyl, hydroxyl and/or ammonium radicals, or a carboxyl-bound amino acid or oligopeptide residue, M and M' hydrogen, an alkali metal, alkaline earth metal or ammonium ion and B a $C_1$-$C_4$-alkylcarbonyl radical possibly substituted one or more times by carboxyl, hydroxyl and/or ammonium radicals, as well as $R^1$ - $R^7$ have the above-given meanings.

**3.** Process for the preparation of colourless and water-soluble compounds according to claim 1, characterised in that, in per se known manner, one then when

a) A is different from Z and A is the same as B, reacts compounds of the general tautomeric general formulae IIa and IIb

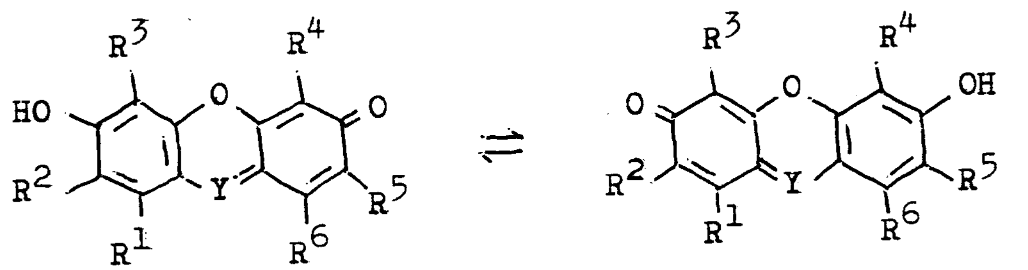

(IIa) (IIb)

in which $R^1$ - $R^6$ have the above-given meanings and Y signifies nitrogen or the group N→O, with

X - Z (III)

whereby Z has the above-given meaning and X signifies a reactive group, and reduces the resorufin derivative resulting as intermediate product of the tautomeric formulae IVa and IVb

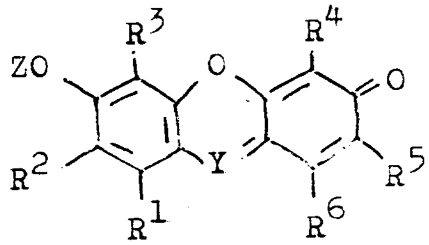

(IVa)

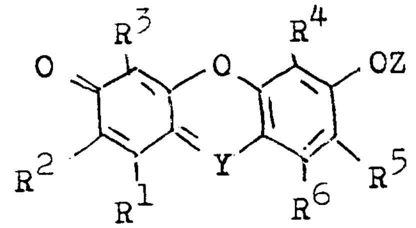

(IVb)

in which $R^1$- $R^6$, Y and Z have the above-given meaning, and acylates by means of

X - B (V)

whereby B has the above-given meaning and X signifies a reactive group; when

b) A is the same as Z and A is different from B, reduces compounds of the general tautomeric formulae IIa and IIb, in which $R^1$ - $R^6$ and Y have the above-given meaning, and acylates by means of

X - B (V)

whereby B has the above-given meaning and X signifies a reactive group, subsequently selectively hydrolyses the ester bond and then reacts with

X - A (VI)

whereby A and X have the above-given meaning, or when
c) A, B and C are all different, reacts compounds of the general tautomeric formulae IIa and IIb, in which $R^1$ - $R^6$ have the above-given meaning and Y represents the group N→O, with

X - A' (VII)

whereby A' has the meaning given above for A or represents a phenol protective group and X signifies a reactive group, subsequently reduces and exhaustively acylates with

X - B (V)

whereby B has the above-given meaning and X represents a reactive group, selectively hydrolyses the last-formed ester bond, reacts with

X - Z (III)

whereby Z has the above-given meaning and X represents a reactive group, and possibly, when A' represents a phenol protective group, removes this and acylates with

X - A (VI)

whereby A has the above-given meaning and X represents a reactive group.

**4.** Use of compounds according to claim 1 for the determination of hydrolases.

**5.** Reagent for the determination of hydrolases, containing one or more chromogenic and/or fluorogenic substrates, a suitable buffer system, as well as possibly further conventionally used reagents and adjuvants, characterised in that, as chromogenic and/or fluorogenic substrates, it contains compounds according to claim 1 or 2, we well as an oxidising agent,

**6.** Reagent according to claim 6, characterised in that, as oxidising agent, it contains $K_3[Fe(CN)_6]$, perborate, atomospheric oxygen, bilirubin oxidase or hydrogen peroxide/peroxidase.

**Revendications**

**1.** Composés incolores et hydrosolubles de formule générale I

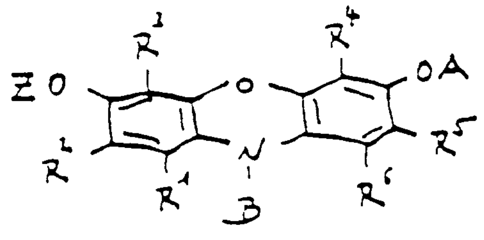

(I)

dans laquelle

| | |
|---|---|
| Z | représente un reste d'acide organique ou inorganique ou un reste sucre, |
| A | représente un reste d'acide organique ou inorganique, |
| B | représente un reste d'acide organique et |
| $R^2$ et $R^5$, | qui peuvent être identiques ou différents, représentent un atome d'hydrogène, d'halogène ou un groupe alkyle en $C_1$-$C_5$, |
| $R^1$, $R^3$, $R^4$ et $R^6$, | qui peuvent être identiques ou différents, représentent un atome d'hydrogène, d'halogène, un groupe cyano, alkyle en $C_1$-$C_5$, alcoxy en $C_1$-$C_5$, carboxy, alcoxycarbonyle en $C_1$-$C_5$, carboxy(alkyle en $C_1$-$C_5$),alcoxy($C_1$-$C_5$)carbonyl-alkyle($C_1$-$C_5$) ou un groupe carboxamido éventuellement substitué une ou deux fois, ou le groupe |

$-COO-(CH_2CH_2O)_n-R^7$

dans lequel

$R^7$ représente un atome d'hydrogène ou un groupe alkyle en $C_1$-$C_5$ et n vaut un nombre entier de 1 à 4,

où

$R^6$ peut représenter en outre un groupe sulfo ou nitro, et où

A, B et Z ne représentent pas simultanément un groupe acyle identique.

**2.** Composés selon la revendication 1, caractérisés en ce que

Z représente $PO_3MM'$, $SO_3M$, un reste d'acide alcanecarboxylique, d'acide aminé ou d'oligopeptide lié à un groupe carboxyle, un reste sucre,

A représente $PO_3MM'$, $SO_3M$, un reste alkyle($C_1$-$C_5$)carbonyle, qui est éventuellement substitué une ou plusieurs fois par un reste carboxyle, hydroxyle et/ou ammonium, ou un reste d'acide aminé ou d'oligopeptide lié à un groupe carboxyle,

M et M' représentent un atome d'hydrogène, un ion alcalin, alcalinoterreux ou ammonium, et

B représente un reste alkyle($C_1$-$C_7$)carbonyle éventuellement substitué une ou plusieurs fois par un reste carboxyle, hydroxyle et/ou ammonium, et

$R^1$-$R^7$ ont les significations mentionnées ci-dessus.

**3.** Procédé de préparation de composés incolores et hydrosolubles selon la revendication 1, caractérisé en ce que, de manière connue en soi, lorsque

a) A est différent de Z et A est identique à B

on fait réagir des composés de formules générales tautomères II a et II b

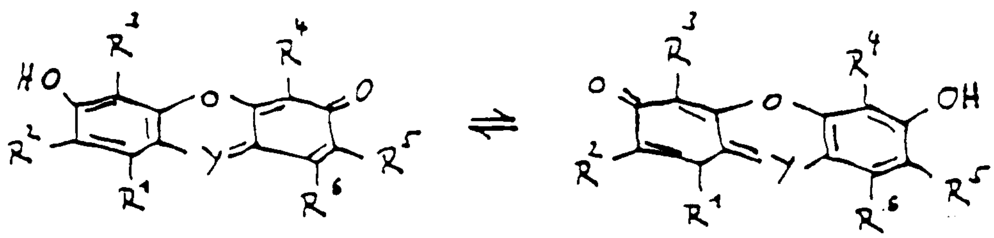

(II a) (II b)

dans lesquelles

$R^1$-$R^6$ ont les significations mentionnées ci-dessus et

Y représente un atome d'azote ou le groupe N→O, avec

X-Z (III)

où

Z a la signification mentionnée ci-dessus, et

X représente un groupe réactif, et on réduit le dérivé de résorufine, formé en tant que produit intermédiaire, répondant aux formules tautomères IV a et IV b

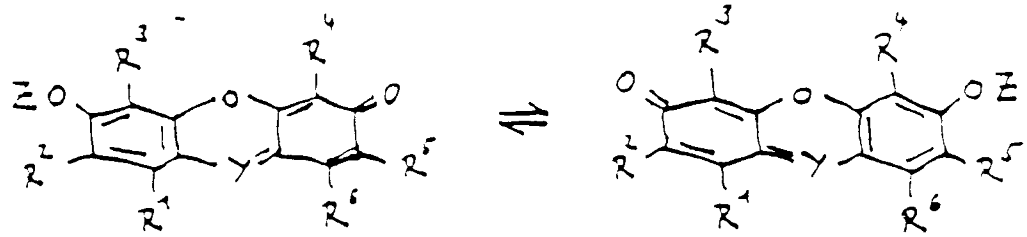

(IV a) (IV b)

dans lesquelles
$R^1$-$R^6$, Y et Z ont les significations mentionnées plus haut, et au moyen de

X-B (V)

où
B a la signification mentionnée ci-dessus et
X représente un groupe réactif, on l'acyle,
lorsque
b) A est identique à Z et A est différent de B
on réduit des composés de formules générales tautomères II a et II b
dans lesquelles
$R^1$-$R^6$ et Y ont les significations mentionnées plus haut et
au moyen de

X-B (V)

où
B a la signification mentionnée ci-dessus et
X représente un groupe réactif, on les acyle, puis on hydrolyse sélectivement la liaison ester et ensuite, on fait réagir avec

X-A (VI)

où
A et X ont les significations mentionnées ci-dessus
ou lorsque
c) A, B et Z sont tous différents,
on fait réagir des composés de formules générales tautomères II a et II b
dans lesquelles
$R^1$-$R^6$ ont les significations mentionnées ci-dessus et
Y représente le groupe N→O, avec

X-A' (VII)

où
A' a la signification mentionnée ci-dessus pour A ou représente un groupe protecteur de phénol, et
X représente un groupe réactif,
puis on les réduit et avec

X-B (V)

où
B a la signification mentionnée ci-dessus et
X représente un groupe réactif, on les acyle complètement,
on hydrolyse sélectivement la liaison ester formée en dernier, on les fait réagir avec

X-Z (III)

où
Z a la signification mentionnée ci-dessus, et
X représente un groupe réactif,
et éventuellement ensuite, lorsque A' représente un groupe protecteur de phénol, on élimine celui-ci et on acyle avec

X-A (VI)

où
A a la signification mentionnée ci-dessus et

X représente un groupe réactif.

**4.** Utilisation de composés selon la revendication 1, pour la détermination d'hydrolases.

**5.** Réactif pour la détermination d'hydrolases, contenant un ou plusieurs substrats chromogènes et/ou fluorogènes, un système tampon approprié ainsi qu'éventuellement d'autres réactifs et adjuvants usuels, caractérisé en ce que, en tant que substrat chromogène et/ou fluorogène, il contient des composés selon la revendication 1 ou 2, ainsi qu'un agent oxydant.

**6.** Réactif selon la revendication 6, caractérisé en ce que, en tant qu'agent oxydant, il contient du $K_3$[Fe-(CN)$_6$], du perborate, de l'oxygène de l'air, de la bilirubinoxydase ou du peroxyde d'hydrogène/peroxydase.